# EUROPEAN PATENT APPLICATION

(11) **EP 2 123 285 A1**
(43) Date of publication of application: **25.11.2009**
(21) Application number: 08305187.0
(22) Date of filing: 21.05.2008
(51) Int. Cl.: A61K 31/7076, A61P 35/00

(54) **Nucleosidic phosphoantigens for use in VGAMMA9DELTA2 T cell-mediated therapy**

(71) Applicant: INSERM (Institut National de la Santé et de la Recherche Médicale), 75654 Paris Cedex 13 (FR)
(72) Inventor: The designation of the inventor has not yet been filed
(74) Representative: Cabinet Plasseraud

(57) **Abstract**

The present invention relates to nucleosidic phosphoantigens for the treatment of the human or animal body by therapy. The invention also relates to a method for stimulating Vγ9Vδ2 T cell-mediated immune response, which comprises the step of administering to a subject in need thereof a therapeutically effective amount of nucleosidic phosphoantigens.

## Description

### FIELD OF THE INVENTION

The present invention relates to nucleosidic phosphoantigens for use in Vγ9Vδ2 T cell-mediated therapy.

### BACKGROUND OF THE INVENTION

The majority of human peripheral T cells of the γδ type express an invariant T cell receptor (TCR) comprising Vγ9 and Vδ2 variable regions. These usually represent 1 to 10% of circulating T lymphocytes, expand and release inflammatory cytokines in response to diverse infectious agents such as mycobacteria, bacteria and parasites (Chen, Z.W. & Letvin, Microbes Infect 5, 491-498, 2003). They also display cytotoxicity towards diverse tumor cells including laboratory tumor lines (Daudi Burkitt's lymphoma, RPMI-8226 myeloma) and multiple cancer lines from patients with, among others, lung, prostate, liver or kidney cancers (Hayday, A.C., Annu Rev Immunol 18, 975-1026, 2000; Kabelitz, D. et al., Int J Cancer 112, 727-732, 2004; Dieli, F. et al., Cancer Res 67, 7450-7457, 2007).
Antigenic molecules which stimulate specifically Vγ9Vδ2 T cells in a TCR-dependent manner have been purified from mycobacteria and belong to a class of antigens termed phosphoantigens because they consist of a short alkyl chain (usually 5 carbon atoms) and a terminal pyrophosphate group (Constant, P. et al., Science 264, 267-270, 1994; Poquet, Y. et al., Eur J Immunol 26, 2344-2349, 1996; Morita, C.T. et al., Immunol Rev 215, 59-76, 2007).
The most powerful natural antigen that has been reported is 4-hydroxy-3-methyl-but-2-enyl pyrophosphate (HMBPP), a metabolite produced by many pathogens through the deoxyxylulose pathway (also called Rhomer pathway), an alternative bacterial route which leads to the isoprenoid precursor isopentenyl pyrophosphate (IPP) (Altincicek, B. et al., J Immunol 166, 3655-3658, 2001; Puan, K.J. et al., Int Immunol, 2007; Eberl, M. et al., FEBS Lett 544, 4-10, 2003).
These pyrophosphorylated antigens can directly activate Vγ9Vδ2 T cells and do not seem to require to be presented by specialized antigen presenting cells or to be displayed on MHC antigens (Morita, C.T. et al., Immunol Rev 215, 59-76, 2007; Bukowski, J.F. et al., J. Immunol. 154, 998-1006, 1995).
Nucleosidic derivatives of these phosphoantigens have also been purified from mycobacteria (Constant, P. et al., Science 264, 267-270, 1994; Poquet, Y. et al., Eur J Immunol 26, 2344-2349, 1996).
Multiple synthetic antigens analogous to pyrophosphorylated antigens as well as nucleosidic variants have been studied and some of them proved to be powerful stimulants of Vγ9Vδ2 T cells (Morita, C.T. et al., J Immunol 167, 36-41, 2001; Tanaka, Y. et al., Proc Natl Acad Sci U S A 91, 8175-8179, 1994; Tanaka, Y. et al., Nature 375, 155-158, 1995).
Tumor cells also produce weakly activatory phosphoantigens through the mevalonate pathway. The major stimulatory compound was found to be IPP (isopentenyl pyrophosphate) which is overproduced in many tumors following hyper-activation of the mevalonate pathway (Gober, H.J. et al. J Exp Med 197, 163-168 (2003); Thompson, et al. J Bone Miner Res 19, 278-288 (2004)). Other phosphoantigens have been generally disclosed in several patent applications, notably in WO 2004/050096, but their activity and activation pathway has not yet been fully evidenced.
IPP can also accumulate in cells after treatment with aminobisphosphonates, a class of drugs which inhibit the mevalonate pathway enzyme farnesyl pyrophosphate synthase. Treatment with aminobisphosphonates causes intracellular accumulation of IPP and its isomer dimethyl allyl pyrophosphate (DMAPP) so that they become sensitive to Vγ9Vδ2 T cell cytolytic activity. Although phosphoantigen recognition by Vγ9Vδ2 T cells is TCR-dependent (Bukowski, J.F. et al. J. Immunol. 154, 998-1006, 1995), the precise mechanism underlying this reactivity is unknown and there is yet no direct proof of phosphoantigen binding to the TCR (Allison, T.J. et al., Mol Immunol 38, 1051-1061, 2002).
Similarly, there is no clear evidence that endogenous pyrophosphorylated antigens are released by tumor cells although they can clearly accumulate intracellularly (Gober, H.J. et al. J Exp Med 197, 163-168, 2003).
We have previously reported that tumor cell lines which stimulate Vγ9Vδ2 T cells express the ecto-F₁-ATPase complex on the cell surface (Scotet, E. et al. Immunity 22, 71-80, 2005). Moreover, a purified form of this complex is able to specifically activate this lymphocyte subset. On the cell surface, this complex is closely associated to MHC class I antigens which regulate Vγ9Vδ2 T cell activation (Vantourout, P. et al., Mol Immunol 45, 485-492, 2008). Ecto-F₁-ATPase is the first direct ligand known for the Vγ9Vδ2 TCR. However, ecto-F₁-ATPase is expressed on several normal tissues and it seems likely that ecto-F₁-ATPase expression and phosphoantigen production must be combined to produce Vγ9Vδ2 T cell activation (Champagne, E. et al., Curr Opin Lipidol 17, 279-284, 2006).

Thereby, extensive work has previously documented the stimulation of Vγ9Vδ2 lymphocytes in a TCR-dependent manner by various stimuli including soluble pyrophosphorylated antigens, tumor cells, aminobisphosphonate-treated cells or purified ecto-F₁-ATPase but a functional link between these stimulations has still remained speculative. Available data indicate that aminobisphosphonate treatment of cells stimulates the mevalonate pathway in a way which is similarly observable with spontaneously activatory tumors such as the Daudi cell line. However, this stimulatory activity is resistant to phosphatases and thus strikingly differs from the direct stimulation of T cells by pyrophosphorylated antigens. Previous experiments indicated that these antigens can activate the TCR without requirement for specialized presenting cell. Although bacterial phosphoantigens display strong stimulatory activity, there is no proof that these phosphoantigens are released in the extracellular environment in the case of intracellular infections which are the hallmark of Vγ9Vδ2 expansions in pathophysiological conditions. Moreover, these antigens are highly sensitive to ubiquitous phosphatases which presumably render their half-life very short. Tumor cells produce pyrophosphorylated antigens which are poor stimulants (as compared to bacterial antigens), are sensitive to phosphatases and do not seem to be released extracellularly. Thus a direct involvement of pyrophosphorylated antigens in physiological conditions is questionable.

Moreover, it has been reported that, even if a single injection of phosphoantigens leads to a strong activation of Vγ9Vδ2 T cells, successive infusions of phosphoantigens induce less vigorous expansions, evidencing a progressive exhaustion of the response (Sicard et al., J Immunol 175, 5471-5480, 2005). This exhaustion of the Vγ9Vδ2 T cells activation certainly results from the non-physiological activation pathway used by phosphoantigens to activate Vγ9Vδ2 T cells, since phosphoantigens cannot be pulsed on presenting cells (Tanaka, Y. et al. Proc Natl Acad Sci U S A 91, 8175-8179, 1994; Morita, C.T. et al. Immunity 3, 495-507, 1995).

There is thus a need for Vγ9Vδ2 T cell activators having increased potency and half-life and not inducing an exhaustion of the Vγ9Vδ2 T cells activation after successive infusions.

### SUMMARY OF THE INVENTION

The present invention relates to a compound of general formula (I) for the treatment of the human or animal body by therapy: 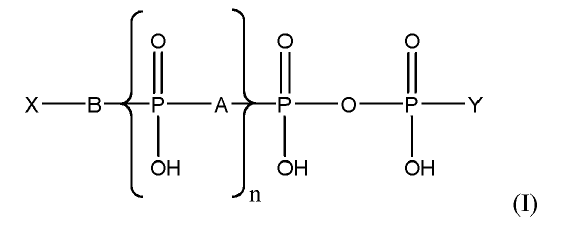 wherein
- n is an integer from 1 to 5;
- B is present or not and if present is selected from the group consisting of O, NH, CHF, CF₂ and CH₂;
- Y is a nucleoside;
- X is a linear or branched C₁-C₅ alkyl or C₂-C₅ alkylenyl, substituted or not by at least one halogen atom, an hydroxyl group or an epoxide group; and
- if n is 1, A is present or not and if present is selected from the group consisting of O, NH, CHF, CF₂ and CH₂; and
- if n is 2, 3, 4 or 5, A is independently present or not and if present is independently selected from the group consisting of O, NH, CHF, CF₂ and CH₂,
   or a pharmaceutically acceptable salt thereof,
   wherein said compound
   i) is cleavable by a nucleotide pyrophosphatase, and
   ii) can bind to F1-ATPase.

In another aspect, the present invention relates to a compound according to the invention for stimulating Vγ9Vδ2 T cell-mediated immune response.
In a particular aspect, the present invention relates to a compound according to the invention for treating a disease selected for the group consisting of cancer, infectious disease, autoimmune disease and inflammatory disease

The present invention also relates to a method for stimulating Vγ9Vδ2 T cell-mediated immune response, wherein said method comprises the step of administering to a subject in need thereof a therapeutically effective amount of a compound according to the invention.
The present invention also relates to a method for treating a disease selected for the group consisting of cancer, infectious disease, autoimmune disease and inflammatory disease, wherein said method comprises the step of administering to a subject in need thereof a therapeutically effective amount of a compound according to the invention.

In still another aspect, the present invention relates to a method for pulsing human antigen-presenting cells with a compound according to the invention, said method comprising the step of culturing antigen-presenting cells *ex vivo* with at least one of the compound according to the invention.

The invention also relates to an antigen-presenting cell obtainable by the method as defined above, for the treatment of the human or animal body by therapy.
The invention also relates to an antigen-presenting cell obtainable by the method as defined above, for treating a disease selected for the group consisting of cancer, infectious disease, autoimmune disease and inflammatory disease.

The invention further relates to a vaccine comprising an antigen or a combination of antigens and a compound according to the invention.
Typically, the invention relates to a vaccine for treating a disease selected from the group consisting of cancers or infectious diseases.

The present invention also relates to a vaccine kit comprising
a) an antigen or a combination of antigens, and
b) a compound according to the invention.

The present invention also provides a method for immunizing a subject against a cancer or an infectious disease comprising administering to said subject
(i) an antigen or combination of antigens, and
(ii) a compound according to the invention.

### DETAILED DESCRIPTION OF THE INVENTION

By "a compound according to the invention", it is meant a compound of formula (I) as defined above or a pharmaceutically acceptable salt thereof. Indeed, *in vitro* or *in vivo*, the compound may be in its anionic form (totally or partially), depending on the pH.
By "treating" or "treatment", as used herein, it is meant reversing, alleviating, inhibiting the progress of, or preventing the disease to which such term applies.
By a "subject" it is meant a human or a warm-blooded animal.
By a "therapeutically effective amount" of a compound according to the invention it is meant a sufficient amount to treat a disease at a reasonable benefit/risk ratio applicable to any medical treatment. It will be understood, however, that the total daily usage of a compound according to the invention will be decided by the attending physician within the scope of sound medical judgment. The specific therapeutically effective dose level for any particular subject in need thereof will depend upon a variety of factors including the stage of the disease being treated, the age, body weight, general health, sex and diet of the subject, the time of administration, route of administration, the duration of the treatment; drugs used in combination or coincidental with the compound according to the invention and like factors well known in the medical arts. For example, it is well known within the skill of the art to start doses of the compound at levels lower than those required to achieve the desired therapeutic effect and to gradually increase the dosage until the desired effect is achieved.
By "antigen", it is meant a substance that prompts the generation of antibodies and can cause an immune response. Typically, the antigen is a tumor antigen, a pathogen antigen. The antigen may for example be a polypeptide, a peptide, a nucleic acid encoding a antigenic polypeptide, a killed, an inactivated or an attenuated pathogen. Examples of pathogens may be a virus, a bacterium, a yeast or parasite.

The present invention relates to a compound of general formula (I) for the treatment of the human or animal body by therapy: 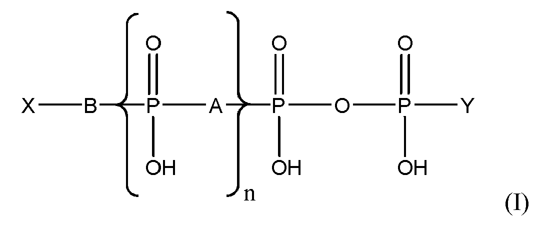 wherein
- n is an integer from 1 to 5;
- B is present or not and if present is selected from the group consisting of O, NH, CHF, CF₂ and CH₂;
- Y is a nucleoside;
- X is a linear or branched C₁-C₅ alkyl or C₂-C₅ alkylenyl, substituted or not by at least one halogen atom, an hydroxyl group or an epoxide group; and
- if n is 1, A is present or not and if present is selected from the group consisting of O, NH, CHF, CF₂ and CH₂; and
- if n is 2, 3, 4 or 5, A is independently present or not and if present is independently selected from the group consisting of O, NH, CHF, CF₂ and CH₂,
   or a pharmaceutically acceptable salt thereof,
   wherein said compound
   i) is cleavable by a nucleotide pyrophosphatase, and
   ii) can bind to F1-ATPase.

Typically, the presence of phosphoester bonds renders the compound according to the invention cleavable by a nucleotide pyrophosphatase (NPP) such as NPP1-7 which can be present on the membrane of cells or secreted by cells and are described in Stefan, Trends Biochem Sci, 30, 542-550, 2005. Alternatively, purified crotale venom pyrophoshatase can be used to cleave the compound *in vitro.* The hydrolysis of the compound according to the invention by a nucleotide pyrophosphatase leads to the liberation of a nucleoside mono, di or poly-phosphorylated or not phosphorylated, and of a phosphoantigen of formula: X-B-PPi, wherein "PPi" represents a pyrophosphate or an analog thereof, wherein said phosphoantigen is able to stimulate Vγ9Vδ2 T cells. The ability of the phosphoantigen to stimulate Vγ9Vδ2 T cells can be measured as disclosed in the experimental section, or by any other method known by the man skilled in the art.

Typically, within the context of the invention, the ability of the compound according to the invention to bind to F1-ATPase can be measured by any known binding method. Binding assays are classical experiments and do not present an undue burden to the man skilled in the art. Moreover, a binding assay suitable for this invention is fully described in the experimental section of this application and, if necessary, can be routinely adapted by the skilled person to other nucleosidic phosphoantigens.

Typically, pharmaceutically acceptable salts of the compound according to the invention have the general formula (I'): 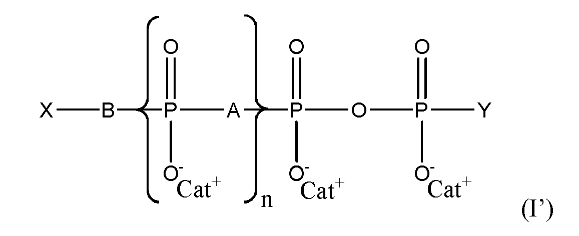 wherein
- Cat⁺ represents one or several, identical or different, organic or mineral cation(s), preferably selected from the group consisting of Na⁺, NH₄⁺, K⁺, Li⁺ or (CH₃CH₂)₃NH⁺;
- n, A, B, Y and X have the same definition as in formula (I).

In an embodiment, the invention relates to a compound according to the invention for stimulating Vγ9Vδ2 T cell-mediated immune response.
The stimulation of the Vγ9Vδ2 T cell-mediated immune response is beneficial, in particular, for treating a disease selected for the group consisting of cancer, infectious disease, autoimmune disease and inflammatory disease.
In an embodiment, the invention relates to a compound according to the invention for treating a disease selected for the group consisting of cancer, infectious disease, autoimmune disease and inflammatory disease.

In one embodiment, the disease is a cancer.
Typically it may be solid tumors. The cancer may for example be selected from the group consisting of lymphoma, kidney, liver, lung and prostate cancer.
In a specific embodiment, said cancer comprises tumor cells expressing on their surface the F₁-ATPase.
Within the context of the present invention, the man skilled in the art is capable to determine by any known method whether the previously mentioned cells express, on their surface, the F₁-ATPase. In particular, a method is fully described in Vantourout et al, Mol. Immunol, 45, 485-492, 2008. Still in the context of this invention, an F₁-ATPase designates any isoforms and orthologs thereof. Determination of said cell surface expression can be performed using a variety of techniques known per se, such as by ligand binding, immunohistochemistry, flow cytometry, etc. Typically, the method comprises incubating the biological sample comprising said cells with a ligand specific for F₁-ATPase, and assessing binding of said ligand to said cell. Typically, the ligand is an antibody or a fragment or derivative thereof (e.g. a Fab or Fab'₂ fragment, a CDR region, a scFv, etc.). The ligand (e.g. antibody) may be labelled, e.g., by fluorescent, enzymatic, luminescent, radioisotope, etc. labels, to facilitate binding determination.

In another embodiment, the disease is an infectious disease. Typically, the disease may be selected from the group consisting of tuberculosis, lepra, malaria, ehrlichiosis and tularaemia.
In another embodiment, the disease is an autoimmune disease or inflammatory disease. Typically, the disease may be selected from the group consisting of multiple sclerosis, rheumatoid arthritis, autoimmune diabetes, lupus erythematosus, inflammatory bowel disease, dilated cardiomyopathy and Takayasu's arteritis.

Typically, in the formula (I) or (I') n is 1, 2, 3, 4 or 5. Preferably, n is 1, 2 or 3.

Typically, B is present and is selected from the group consisting of O, NH, CHF, CF₂ and CH₂. If B is absent, X is directly linked to the phosphorus atom. In one embodiment, B is selected from the group consisting of O, CHF, CF₂ and CH₂. In a specific embodiment, B is selected from the group consisting of O and CH₂. In another embodiment B is O.

Typically, if n is 1, A is present and is selected from the group consisting of O, NH, CHF, CF₂ and CH₂. If A is not present, the two phosphorus atoms are directly linked together. In one embodiment, A is selected from the group consisting of O, CHF, CF₂ and CH₂. In a specific embodiment, A is selected from the group consisting of O and CH₂. In another embodiment A is O.

Typically, if is n is 2, 3, 4 or 5, A is independently present or not and if present is independently selected from the group consisting of O, NH, CHF, CF₂ and CH₂. If an A is not present, the two phosphorus atoms are directly linked together. In one embodiment, A is independently selected from the group consisting of O, CHF, CF₂ and CH₂. In a specific embodiment, A is independently selected from the group consisting of O and CH₂. In another embodiment A is O.

Typically, Y comprises a purine or pyrimidine base linked to a pentose. Examples of Y according to the invention are adenosine (A), guanosine (G), thymine (T), cytidine (C), uridine (U), Xanthine (X) or Hypoxanthine (H) and derivatives thereof. "Derivatives" within the meaning of the invention can be deoxynucleosides, e.g. deoxyadenosine (dA), deoxyguanosine (dG), deoxythymine (dT), deoxycytidine (dC), deoxyuridine (dU), deoxyxantosine (dX) or deoxyinosine (dI). Other derivatives according to the invention are dideoxynucleosides, e.g. dideoxyadenosine (ddA), dideoxyguanosine (ddG), dideoxythymine (ddT), dideoxycytidine (ddC), dideoxyuridine (ddU), ddideoxyxanthosine (ddX) or dideoxyinosine (ddI). Further examples of derivatives are nucleosides wherein at least one hydrogen or one hydroxyl group of the base or of the ribose ring are substituted by another functional group, e.g. SH, S-CH₃, NH₂.
In a preferred embodiment Y is an adenosine or a derivative thereof.

In the context of the present invention, the term "C₁-C₅ Alkyl" means a C₁, C₂, C₃, C₄, or C₅ alkyl. More specifically, it means a group such as methyl, ethyl, propyl, isopropyl, butyl, isobutyl, tert-butyl, pentyl, isopentyl.

The term "C₂-C₅ Alkylenyl" refers to a C₂, C₃, C₄, or C₅ alkyl group as defined hereinabove having at least one unsaturated ethylene bond. More specifically it refers to ethenyl, a propenyl (1-propenyl or 2-propenyl), a 1- or 2- methylpropenyl, a butenyl (1-butenyl, 2-butenyl or 3-butenyl), a methylbutenyl, a 2-ethylpropenyl, a pentenyl (1-pentenyl, 2-pentenyl, 3-pentenyl, 4-pentenyl), and the other isomeric forms thereof.

Typically, X is a linear or branched alkylenyl with one or several double bonds. Preferably, the alkylenyl group has one double bond.
Optionally, said alkenyl group can be the isoform trans (E) or cis (Z), more preferably a trans isoform (E).
Preferably, the alkylenyl group is a (C3-C5) alkylenyl group.
Preferably, said alkylenyl group is substituted by at least one functional group. More preferably, the functional group is selected from the group consisting of a hydroxyl, a halogen, an epoxide. Within the context of the invention, the halogen can be Cl, Br, F or I. Preferably the halogen is Br or F.

In another embodiment, X is a linear or branched C₁-C₅ alkyl or C₂-C₅ alkylenyl, selected from the group consisting of:
1/ 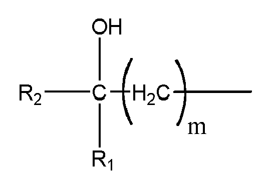
2/ 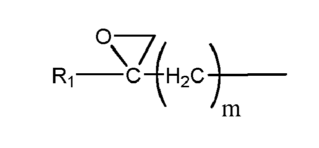
3/ 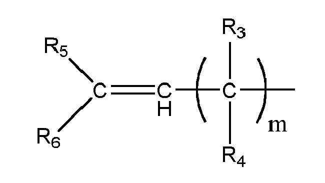 wherein
   - m is an integer from 1 to 3,
   - R1 and R2 are identical or different and selected from the group consisting of H, Cl, Br, F, methyl and ethyl,
   - R3, R4, R5 and R6 are identical or different and selected from the group consisting of H, Cl, Br, F, methyl, ethyl and OH.

In another embodiment, X is isopentenyl, i.e. 3-methylbut-3-enyl.
In another embodiment X is a dimethylallyl or a hydroxydimethylallyl. Typically, X is (E)-4-hydroxy-dimethylallyl.
In still another embodiment, X is butenyl substituted by a group -CH₂OH.
In still another embodiment, X is the (E)-4-hydroxy-3-methyl-but-2-enyl.
In another embodiment, X is 3-(bromomethyl)-3-butanol-1-yl.
In another embodiment, X is 3,4-epoxy-3-methyl-1-butyl.

In one embodiment, the compound according to the invention has the general formula (I) wherein:
- n is from 1 to 3;
- B is present or not and if present is selected from the group consisting of O, NH, CHF, CF₂ and CH₂;
- Y is a nucleoside;
- X is a linear or branched C₁-C₅ alkyl or C₂-C₅ alkylenyl, substituted or not by at least one halogen atom, an hydroxyl group or an epoxide group; and
- if n is 1, A is present or not and if present is selected from the group consisting of O, NH, CHF, CF₂ and CH₂;
- if n is 2 or 3, A is independently present or not and if present is independently selected from the group consisting of O, NH, CHF, CF₂ and CH₂,
   or a pharmaceutically acceptable salt thereof.

In one embodiment, the compound according to the invention has the general formula (I) wherein:
- n is from 1 to 3;
- B is absent;
- Y is a nucleoside;
- X is a linear or branched C₁-C₅ alkyl or C₂-C₅ alkylenyl, substituted or not by at least one halogen atom, an hydroxyl group or an epoxide group; and
- if n is 1, A is present or not and if present is selected from the group consisting of O, NH, CHF, CF₂ and CH₂;
- if n is 2 or 3, A is independently present or not and if present is independently selected from the group consisting of O, NH, CHF, CF₂ and CH₂,
   or a pharmaceutically acceptable salt thereof.

In one embodiment, the compound according to the invention has the general formula (I) wherein:
- n is from 1 to 3;
- B is selected from the group consisting of O, NH, CHF, CF₂ and CH₂;
- Y is a nucleoside;
- X is a linear or branched C₁-C₅ alkyl or C₂-C₅ alkylenyl, substituted or not by at least one halogen atom, an hydroxyl group or an epoxide group; and
- if n is 1, A is selected from the group consisting of O, NH, CHF, CF₂ and CH₂;
- if n is 2 or 3, A is independently selected from the group consisting of O, NH, CHF, CF₂ and CH₂,
   or a pharmaceutically acceptable salt thereof.

In one embodiment, the compound according to the invention has the general formula (I) wherein:
- n is from 1 to 3;
- BisO;
- Y is a nucleoside;
- X is a linear or branched C₁-C₅ alkyl or C₂-C₅ alkylenyl, substituted or not by at least one halogen atom, an hydroxyl group or an epoxide group; and
- if n is 1, A is selected from the group consisting of O, NH, CHF, CF₂ and CH₂;
- if n is 2 or 3, A is independently selected from the group consisting of O, NH, CHF, CF₂ and CH₂,
   or a pharmaceutically acceptable salt thereof.

In one embodiment, the compound according to the invention has the general formula (I) wherein:
- n is from 1 to 3;
- B is selected from the group consisting of O, NH, CHF, CF₂ and CH₂;
- Y is a nucleoside;
- X is a linear or branched C₁-C₅ alkyl or C₂-C₅ alkylenyl, substituted or not by at least one halogen atom, an hydroxyl group or an epoxide group; and
- A is O,
   or a pharmaceutically acceptable salt thereof.

In another embodiment, the compound according to the invention has the general formula (I) wherein:
- n is 1;
- B is O;
- Y is a nucleoside;
- X is a linear or branched C₁-C₅ alkyl or C₂-C₅ alkylenyl, substituted or not by at least one halogen atom, an hydroxyl group or an epoxide group; and
- A is O,
   or a pharmaceutically acceptable salt thereof.

In still another embodiment, the compound according to the invention has the general formula (I) wherein:
- n is 1;
- BisO;
- Y is a nucleoside;
- X is a branched C₃-C₅ alkylenyl; and
- A is O,
   or a pharmaceutically acceptable salt thereof.

In another embodiment, the compound according to the invention has the general formula (I) wherein:
- n is 1;
- B is O;
- Y is a nucleoside;
- X is a branched C₅ alkylenyl; and
- A is O,
   or a pharmaceutically acceptable salt thereof.

In another embodiment, the compound according to the invention has the general formula (I) wherein:
- n is 1;
- B is O;
- Y is a nucleoside;
- X is selected from the group consisting of isopentenyl, dimethylallyl, hydroxydimethylallyl, and (E)-4-hydroxy-3methyl-2-but-2-enyl; and
- A is O,
   or a pharmaceutically acceptable salt thereof.

In another embodiment, the compound according to the invention has the general formula (I) wherein:
- n is 1;
- B is O;
- Y is an adenosine;
- X is isopentenyl; and
- A is O,
   or a pharmaceutically acceptable salt thereof.

In another embodiment, the compound according to the invention has the general formula (I) wherein:
- n is 1;
- B is O;
- Y is an adenosine;
- X is 4-hydroxy-3methyl-2-but-2-enyl; and
- A is O,
   or a pharmaceutically acceptable salt thereof.

In another embodiment, the compound according to the invention is 1-(adenosine-5'-yl)-3-(3-methylbut-3-enyl) triphosphoric diester, or a pharmaceutically acceptable salt thereof. In other words, the compound according to the invention is ApppI or a pharmaceutically acceptable salt thereof.

In still another embodiment, the compound according to the invention is 1-(adenosine-5'-yl)-3-((E)-4-hydroxy-3-methyl-but-2-enyl) triphosphoric diester, or a pharmaceutically acceptable salt thereof.

In another embodiment, the compound according to the invention is 1-(adenosine-5'-yl)-3-((E)-4-hydroxy-dimethylallyl) triphosphoric diester, or a pharmaceutically acceptable salt thereof.

In still another embodiment, the compound according to the invention is 1-(adenosine-5'-yl)-3-(3-(bromomethyl)-3-butanol-1-yl) triphosphoric diester, or a pharmaceutically acceptable salt thereof.

In still another embodiment, the compound according to the invention is 1-(adenosine-5'-yl)-3-(3,4-epoxy-3-methyl-1-butyl) triphosphoric diester, or a pharmaceutically acceptable salt thereof.

The compounds of the invention may be produced according to various techniques known per se in the art, some of which being disclosed in WO 00/12516, WO 00/12519 and WO 03/009855. In particular, the compounds of the invention may be produced according to the methods disclosed in WO 04/050096. In another embodiment, the compounds of the invention are produced by a method adapted from Ryu et al., Org. Lett. 5, 4713-4715, 2003, as described in the experimental section.

In another aspect, the present invention relates to a method for stimulating Vγ9Vδ2 T cell-mediated immune response which comprises the step of administering to a subject in need thereof a therapeutically effective amount of a compound according to the invention.

The present invention also relates to a method for treating a disease selected for the group consisting of cancer, infectious disease, autoimmune disease and inflammatory disease, wherein said method comprises the step of administering to a subject in need thereof a therapeutically effective amount of a compound according to the invention.

In still another aspect, the present invention relates to a method for pulsing human antigen-presenting cells with a compound according to the invention, said method comprising the step of culturing antigen-presenting cells (APCs) *ex vivo* with at least one of the compound according to the invention.
The APCs can be prepared from various tissues such as peripheral blood, umbilical cord blood, bone marrow cells and epidermis, as described for example in US 7,029,671. The APCs may be prepared by adding substance(s) such as SCF, IL-1, IL-3, TNF-α and Flk2/Flt3 ligand or a monocyte conditioned medium (MCM) in addition to GM-CSF and IL-4. Moreover, the APCs may also be pulsed with tumor antigens or pathogen antigens.
In a preferred embodiment, APCs are dendritic cells.

The invention also relates to antigen-presenting cells obtainable by the method as defined above, for the treatment of the human or animal body by therapy.
In a specific embodiment, the invention relates to antigen-presenting cells obtainable by the method as defined above, for treating a cancer or an infectious disease.
In an embodiment, the invention relates to a method which comprises the step of administering to a subject in need thereof a therapeutically effective amount of autologous or histocompatible antigen-presenting cells pulsed with a compound according to the invention. By "autologous cells" it is meant cells that are collected from a subject and transferred back to that same subject.

The invention further relates to a vaccine comprising an antigen or a combination of antigens and a compound according to the invention.
Typically, the invention relates to a vaccine for treating a disease selected from the group consisting of cancers or infectious diseases.
In one embodiment, the disease is a cancer.
For example, it may be solid tumors.
The cancer may for example be selected from the group consisting of lymphoma, kidney, liver, lung and prostate cancer.
In a specific embodiment, said cancer comprises tumor cells expressing on their surface the F₁-ATPase.
In another embodiment, the disease is an infectious disease. Typically, the disease may be selected from the group consisting of tuberculosis, lepra, malaria, ehrlichiosis and tularaemia.
Depending on the disease to be treated, said antigen or combination of antigens is a tumor antigen or a combination of tumor antigens or a pathogen antigen or a combination of pathogen antigens.

Indeed, the compound according to the invention may be used as adjuvant. Accordingly, the present invention discloses methods and compositions for stimulating Vγ9Vδ2 T cell-mediated immune response, involving the conjoint immunization of the human or animal body with (i) a composition comprising an antigen and (ii) a compound according to the invention.

The present invention also relates to a vaccine kit comprising
a) an antigen or a combination of antigens, and
b) a compound according to the invention.
   The two elements of the vaccine kit may be uses conjointly or sequentially in a method of vaccination of a subject in need thereof.
   Typically, the antigen or combination of antigens (a) and the compound according to the invention (b) are in the form of solutions for injection. In one embodiment, the kit comprises containers with the solutions for injection and syringes. Alternatively, the container is a syringe. Any other pharmaceutical forms are encompassed by the present invention and the man skilled in the art can routinely adapt the pharmaceutical form to the disease to be treated.

The present invention also provides a method for immunizing a subject in need thereof against a cancer or an infectious disease, comprising administering to said subject
(i) an antigen or combination of antigens, and
(ii) a compound according to the invention.

Further aspects and advantages of this invention will be disclosed in the following figures and examples, which should be regarded as illustrative and not limiting the scope of this application.

### BRIEF DESCRIPTION OF THE FIGURES

**Figure 1****.** Structure of ApppI and IPP. The cleavage sites by nucleotide pyrophosphatase (NPP) and apyrase (Apyr) are indicated.

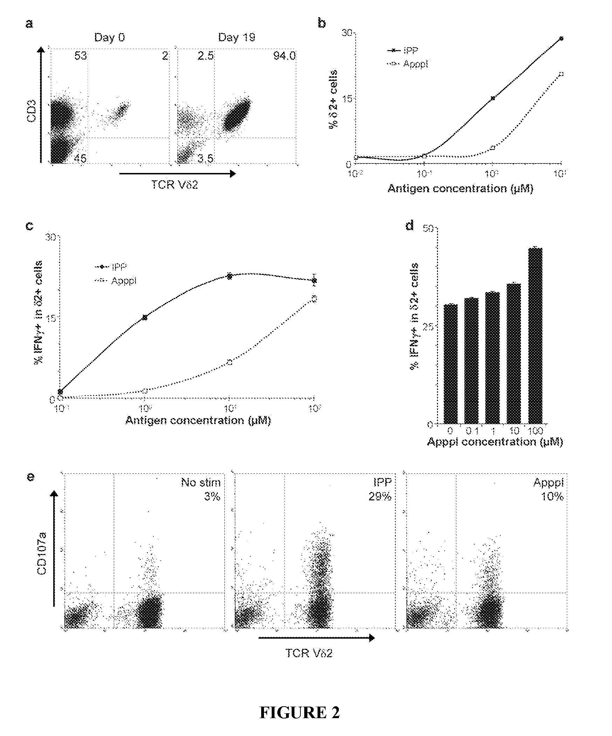

**Figure 2****.** Activation of Vγ9Vδ2 T cells by ApppI. (a,b) PBL were stimulated with phosphoantigens and cells were grown in the presence of IL-2 before staining with anti-CD3-PE and anti-TCRVδ2-FITC antibodies. (a) Comparative percentages of Vδ2⁺ T cells in PBLs before and after in vitro culture (19 days) with ApppI (5µM). Numbers indicate the percentage of cells in each quadrant. (b) Comparative efficiency of Vδ2 T cell amplification 1 week after PBL stimulation with IPP or ApppI. (c) Comparative cytokine production following PBL stimulation with IPP and ApppI. Cells were stained for cell surface TCRVδ2 and intracellular IFNγ. Data are expressed as the mean of triplicate cultures +/- S.E.M. (d) PBL were stimulated in the presence of IPP (10µM) plus ApppI at the indicated concentrations to reveal a putative inhibition of IPP response by ApppI and then stained as in (c). Results are expressed as the mean of triplicate cultures +/- S.E.M. (e) A polyclonal Vγ9Vδ2 T cell line was stimulated with the indicated antigens in the presence of anti-CD 107a-PE antibody to measure the activation of the cytolysis machinery. Cells were then washed and stained for TCRVδ2. The numbers indicate the percentage of CD107a⁺ cells in Vδ2⁺ cells. Data are representative of triplicate stimulation experiments.
**Figure 3****.** Effect of phosphatases and the pyrophosphatase inhibitor PPADS on ApppI and IPP stimulatory activity.
   A polyclonal Vγ9Vδ2 T cell line was stimulated with IPP or ApppI at the indicated concentrations, and expression of CD107a was monitored as in Fig.2e. All data are expressed as the mean of triplicate stimulation experiments +/- S.E.M. (a) Effect of NPP (0.02U/mL) on IPP (10µM) and ApppI stimulations. (b) Individual and combined effects of apyrase (0.2U/mL) and NPP (0.02U/mL) on IPP (10µM) and ApppI (10µM) stimulations. (c) Effect of the nucleotide pyrophosphatase inhibitor PPADS.

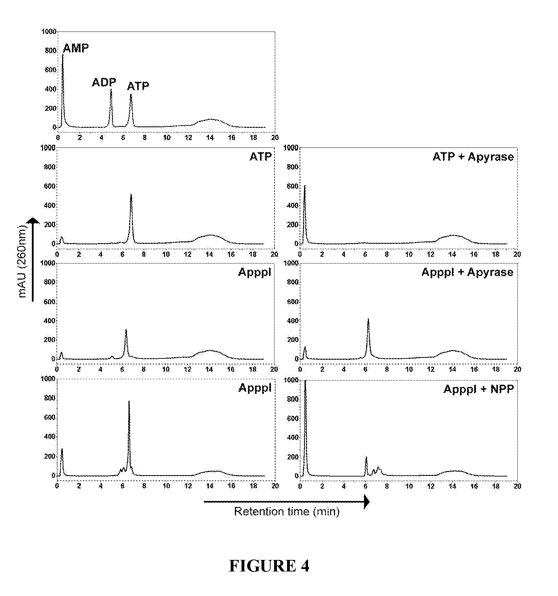

**Figure 4****.** HPLC analysis of ApppI sensitivity to apyrase and nucleotide pyrophosphatase. ATP and ApppI were treated with the indicated enzyme and resulting nucleotides were separated by HPLC as described in the material and methods section. Top panel: mixture of ATP, ADP and AMP to show their relative retention time. Left and right panels are corresponding mock-treated and enzyme-treated samples.

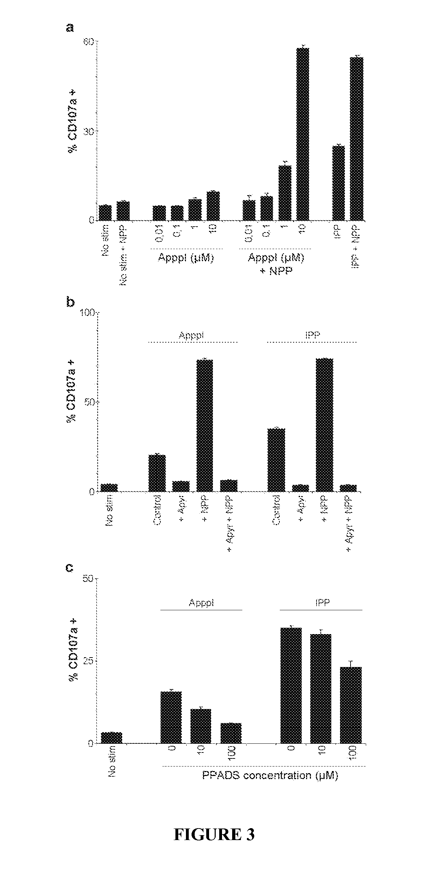

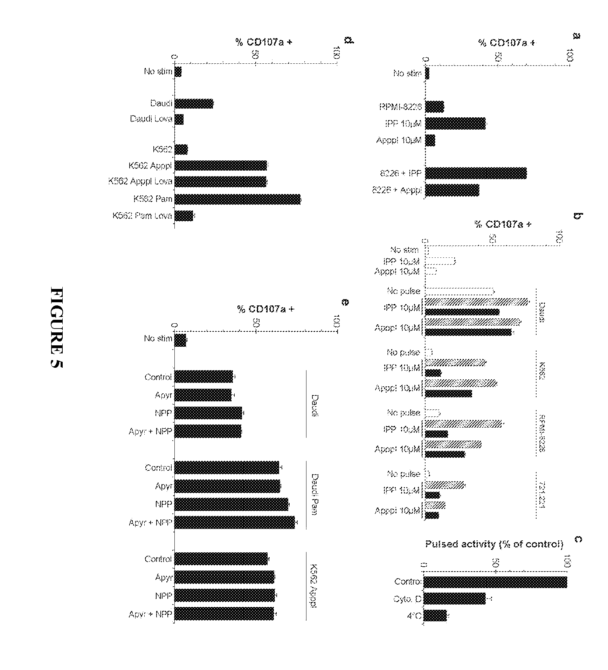

**Figure 5****.** Effect of antigen presentation on ApppI response. A polyclonal Vγ9Vδ2 T cell line was stimulated as indicated and CD107a expression was monitored as in Fig.3 on Vδ2⁺ cells. All data are expressed as mean of triplicate stimulation experiments +/- S.E.M. (a) Stimulation with IPP, ApppI, RPMI-8226 cells (1:1 ratio) or RPMI-8226 plus phosphoantigens. (b) Indicated tumor cell lines were pulsed overnight with the indicated antigens. Before addition of T cells, either the cell supernatant was simply removed by centrifugation (hatched bars) or cells were washed extensively (closed bars). Open bars represent controls (no stimulation, phosphoantigens alone or non-pulsed tumor cells). (c) RPMI-8226 cells were pulsed with ApppI (2h, 10µM) either at 37°C (Control), 4°C, or at 37°C after a 2h pre-treatment with cytochalasin D (10µM), and washed extensively before addition of Vγ9Vδ2 T cells. (d) Daudi and K562 cells were treated overnight with the indicated compounds, washed and used as stimulators for Vγ9Vδ2 T cells. Lova: lovastatin, 20µM; ApppI: 20µM; Pam: pamidronate, 50µM. (e) Tumor cells were pulsed overnight with pamidronate (50µM) or ApppI (20µM), washed extensively, and polyclonal Vγ9Vδ2 T cells were added along with the indicated enzymes (Apyr: Apyrase, 0.2U/mL, NPP: Nucleotide Pyrophosphatase, 0.02U/mL).

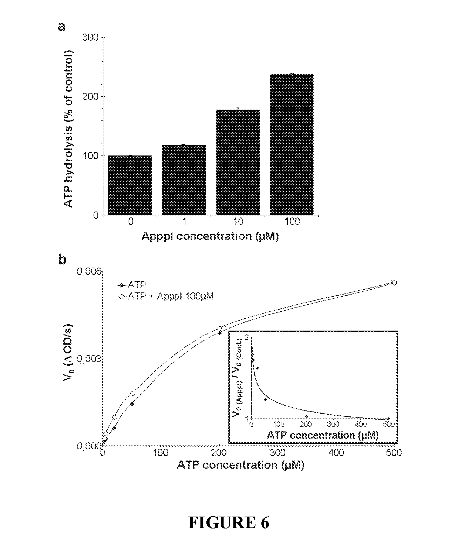

**Figure 6****.** Effect of ApppI on F₁-ATPase hydrolase activity.
   (a) Purified bovine F₁-ATPase was incubated 5min with 1µM ATP and variable concentrations of ApppI. The remaining ATP concentration was then measured by luminescence and the percentage of hydrolysis was calculated (100% corresponds to the amount of hydrolysis obtained in absence of ApppI). Data are expressed as the mean of triplicate hydrolysis experiments +/- S.E.M. (b) The kinetics of ADP production by F₁-ATPase from ATP was measured by the LDH/PK method and the initial rate of hydrolysis was calculated for each indicated ATP substrate concentration, in the presence (V_{0(ApppI)}) or absence (V₀) of ApppI (100µM). Inset: The V_{0(ApppI)}/V₀ ratio was plotted against the ATP concentration to better show the effect of ApppI at low substrate concentrations.

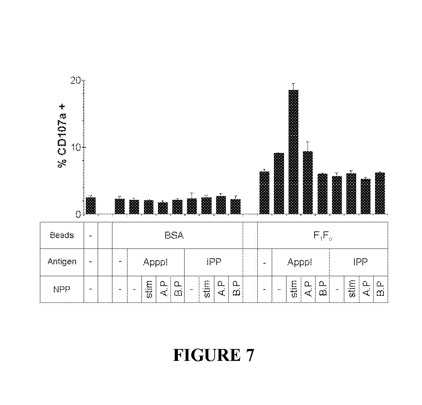

**Figure 7****.** Binding of ApppI to purified F₁Fₒ-ATPase.
   BSA-coated or F₁Fₒ-coated polystyrene beads were incubated for 2h with ApppI or IPP, then washed extensively and used to stimulate Vγ9Vδ2 T cells (1:1 ratio). NPP (0.02U/mL) was added at different steps of the experiment: during stimulation of T cells (stim), in ApppI 30min before pulsing the beads (B.P) or 30 min after the pulse start (A.P). CD107a expression on Vδ2⁺ cells was monitored as in Fig.3. Data are expressed as the mean of triplicate culture experiments +/- S.E.M and the experiment is representative of three.

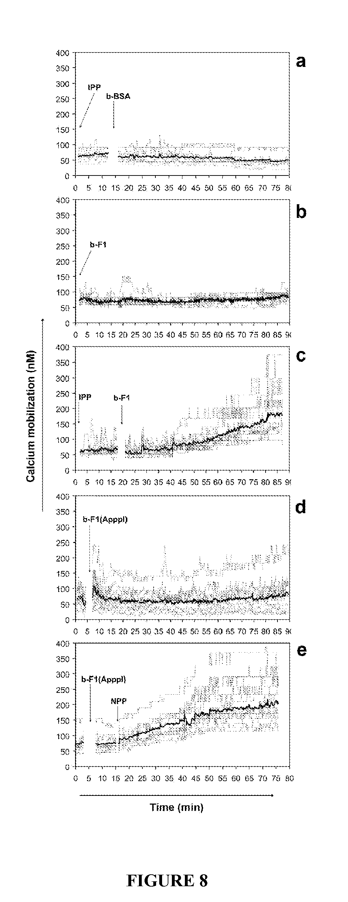

**Figure 8****.** Synergistic effect of F₁-ATPase and phosphorylated antigens on intracellular calcium mobilization. Fura-2-loaded Vγ9Vδ2 T cells were left to adhere to the bottom of Labtek chamber slides at a cell density minimizing cell-cell contacts. IPP (30µM) and nucleotide pyrophosphatase (NPP, 0.02U/mL) were added at the indicated times. Beads carrying F₁-ATPase loaded (b-F1(ApppI)) or not (b-F1) with ApppI and control beads (b-BSA) were added in some experiments and were in large excess to insure that all cells could make multiple contacts with beads. Intracellular calcium concentration was measured by fluorescence microscopy from the ratio of emission (510nm) following excitation at 340 and 380nm and monitored on cells which did not make cell-cell contacts. Data are representative of at least 3 similar recordings for each condition. Each dotted line represents the response of a single cell. Solid lines are the mean response for each condition. a) IPP added at T=0; BSA-coated beads at T=15min; n=10. b) F₁-coated beads at T=0; n=8). c) IPP at T=0 and F₁-coated beads at T=15min; n=13. d) F₁-coated/ApppI-loaded beads at T=0; n=11. e) F₁-coated/ApppI-loaded beads at T=0 and nucleotide pyrophosphatase (NPP) at T=15min; n=14.

### MATERIAL AND METHODS

### Antibodies, F₁-ATPase and other reagents

Anti-TCRVδ2-Fitc (IMMU360) and CD3-PE (UCHT1) were from Beckman-Coulter, anti-IFNγ-PE (25273) from R&D systems, and anti-CD 107a-PE (H4A3) from BD Pharmingen. F₁ and F₁Fₒ complexes from bovine heart mitochondria were kindly provided by Pr. J.E. Walker (MRC, Cambridge, UK). F₁Fₒ complexes solubilized in undecyl-β-D-maltoside were used for immobilization on polystyrene beads whereas F₁ complexes stored as an ammonium sulfate precipitate were used in enzymatic assays. All other reagents were from Sigma Aldrich except Isopentenyl Pyrophosphate (Isoprenoids LC, Tampa, Florida, USA) and ApppI (see below).

### ApppI synthesis

The procedure was adapted from Ryu et al. Org. Lett. 5, 4713-4715 (2003). Briefly, 110 mg (0.2 mmol) of ATP (sodium salt) were converted into the corresponding tetrabutylammonium salt then dissolved in dry acetonitrile (2 mL). To this mixture was added a solution of isopentenyl tosylate (1 eq., 48 mg) in dry acetonitrile (2 mL). The reaction mixture was stirred at room temperature overnight and then concentrated. The oily residue was purified on a DEAE sephadex column using a gradient of triethylammonium bicarbonate buffer (TEAB, pH 6.9, 0.2 to 1M). The required fractions were evaporated under vacuum, and then passed through an ionexchange Dowex (Na⁺ form) column to give ApppI (1-(adenosin-5'-yl) 3-(3-methylbut-3-enyl) triphosphoric diester) as sodium salt with a final yield of 45%. ApppI was stored at -20°C. Aliquots were reconstituted in molecular biology grade water and stored at -80°C. HPLC analysis of reconstituted product revealed the presence of 5-10% ADP (presumably from spontaneous degradation). Although ADP has no effect on T cell stimulations (data not shown) it interfered significantly with F₁-ATPase activity measurements. This contaminating product was efficiently removed by apyrase treatment: ApppI was pre-treated for 1h with 0.2U/mL apyrase. The enzyme was then inactivated by heating at 100°C for 10 minutes. Apyrase inactivation was ascertained by checking the absence of ATP hydrolysis by the apyrase-treated ApppI solution by luminometry (see below).

### Cell culture and pulsing with phosphoantigens

All cell culture reagents were from Invitrogen except human serum (PAA Laboratories) and IL-2 (Sanofi Synthelabo, Toulouse, France). Tumor cell lines were obtained from the ATCC and grown in RPMI 1640 Glutamax supplemented with 10% heat-inactivated FCS, sodium pyruvate, and penicillin/streptomycin or in Hybridoma SFM medium supplemented with sodium pyruvate, penicillin/streptomycin and L-glutamine (Complete SFM) for pulsing experiments since pulsing was less efficient in presence of FCS (data not shown). In this case, cells were washed and incubated with the indicated concentration of IPP or ApppI for 2h or 16h, washed again 4 times, pelleted by centrifugation and used for T cell stimulation assays. For polyclonal Vγ9Vδ2 T cell lines establishment, PBL isolated from buffy coats from healthy donors (Etablissement Français du Sang, Toulouse, France) were stimulated with 5µM phosphoantigen (IPP or ApppI) in RPMI 1640 Glutamax medium supplemented with 10% heat-inactivated human serum (type AB), sodium pyruvate and penicillin/streptomycin. 24h after stimulation, IL-2 was added at a final concentration of 200U/mL. On day 5, IL-2 concentration was raised to 400U/mL. Cells were passed every 2-3 days, maintained at a concentration of 6.10⁵/mL and frozen after 22-24 days of culture, until further use. Using this protocol the cell lines were usually 75-95% Vδ2⁺.

### Proliferation assay

PBL were stimulated as described for polyclonal Vγ9Vδ2 T cell lines establishment, collected at the indicated times and stained for 30min at 4°C with anti-TCRVδ2-Fitc and anti-CD3-PE antibodies (1/25 and 1/50 final dilutions, respectively) in PBS 5% FCS (FACS medium). Data were acquired using a FACScan cytometer (Becton Dickinson) and analyzed with WinMDI 2.9 (TSRI, La Jolla, CA, USA).

### Intracellular cytokine staining

Polyclonal Vγ9Vδ2 T cell lines were stimulated for 5h in 96 U bottom wells plates with the indicated antigens after a brief (30sec) centrifugation to increase cell-cell contact. Brefeldin A (10µg/ml) was added 1h after the initiation. Cells were then washed and stained with anti-TCRVδ2-Fitc antibody (1/25 final dilution) in FACS medium, fixed for 15min with PBS 2% paraformaldehyde (w/v) at room temperature and then permeabilized with saponin buffer (PBS, 10mM HEPES, 5% FCS, 0.1% saponin) for 15min at room temperature. Cells were then stained with anti-IFNγ-PE antibody (1/100 final dilution) in saponin buffer for 30min at 4°C and washed twice in FACS medium. Data were acquired and analyzed as for proliferation assay. Results are expressed as the mean of triplicate microwell cultures +/-S.E.M.

### Degranulation assay for cytolytic function

Experiments were performed as previously described (Betts, M.R. et al. J Immunol Methods 281, 65-78, 2003) with minor modifications. Brefeldin A was omitted. Briefly, polyclonal Vγ9Vδ2 T cell lines (10⁵ cells) were stimulated in 96 U bottom wells plates with the indicated antigens, F₁Fₒ-coated beads or tumor cell lines (1:1 ratio) for 5h after a brief centrifugation, in presence of the anti-CD107a-PE antibody (1/25 final dilution) in a final volume of 100 µL. Cells were washed twice in FACS medium and stained for TCR-Vδ2. Data were acquired and analyzed as for proliferation assay. Results are expressed as the mean of triplicate microwell cultures +/-S.E.M.

### Pulsing ApppI on F₁Fₒ-coated beads and subsequent T cell activation

For immobilization on beads, solubilized F₁Fₒ complexes were diluted to 1/3000 (5µg/mL) in 1 ml of PBS and incubated with 10⁷ 6µm polystyrene beads (Biovalley, Marne-la-Vallée, France) for 4h at room temperature on a rotating wheel. Beads were then pelleted by centrifugation (2min, 12000g), washed and incubated in 1mL of 1% BSA in PBS overnight, washed once with PBS and kept for several days at 4°C in PBS containing 1% BSA. Beads were used in stimulation assays as cultured cell lines. Control beads were prepared similarly except that F₁Fₒ was replaced by BSA. In some experiments the beads were loaded with phosphoantigens: to this end, after washing a suitable aliquot, beads were incubated with ApppI or IPP (50µM) for 2 hours at 37°C in complete SFM medium, washed extensively, and used for Vγ9Vδ2 T cells stimulation. In phosphatase sensitivity assays (Fig.8) NPP or control medium were added to the beads in microculture wells 30min before pulsing the beads or addition of T cells. The beads were then washed and T cells were added with or without supplementation with the same phosphatases. T cell activation was evaluated as described above.

### HPLC analysis of nucleosidic compounds

Reaction mixes containing nucleosidic compounds were adjusted to 200µL with water and products were separated by HPLC (System Gold, Beckman Coulter) on a PL-SAX anion exchange column (1000Å, 5µm, 50x4.6mm from Polymer Labs Varian, Marseille, France) using water (A) and 1M (NH₄)₂HPO₄, pH 3.5 (B) and the following elution gradient: 0-1 min: 0% B; 1-11 min: 0% to 30% B; 11-13 min: 100% B; 13-19min: 0% B. Eluted nucleotides were detected by monitoring UV absorbance at 260nm. In these conditions, adenosine and AMP are not retained on the column and are detected with the elution front. To evaluate phosphatase sensitivity, ApppI and ATP (100µM final concentration) were incubated for 1h at 37°C in 20µL RPMI in the presence of 0.2U/mL apyrase or 0.02U/mL nucleotide pyrophosphatase before analysis.

### Measurement of F₁-ATPase activity

F₁-ATPase complexes were recovered from ammonium sulfate precipitates by centrifugation (17000g, 3min) and dissolved in ATPase assay buffer (10mM HEPES, 150mM NaCl, 5mM KCl, 2mM MgCl₂, pH 7.5). ATP and ADP were measured using two different assays.
For ATP measurements, a bioluminescence assay was used: 100ng F₁-ATPase were incubated at room temperature in 50µL of ATPase assay buffer containing 1µM ATP and the indicated apyrase-treated ApppI concentrations in white 96 wells plates. After 5 minutes, 30µL of reconstituted luciferase from the ATP CLSII Kit (Roche) were added and luminescence was acquired on an Orion luminometer (Berthold Detection Systems).
The kinetics of ADP production resulting from ATP hydrolysis was measured using the lactate dehydrogenase/pyruvate kinase assay and was adapted from Gledhill, J.R. et al., Biochem J 386, 591-598, 2005: 2µg F₁-ATPase were incubated in 200µL of ATPase assay buffer supplemented with phosphoenol pyruvate (500µM), NADH (250µM), lactate dehydrogenase (LDH, 1U) and pyruvate kinase (PK, 5U), with the indicated ATP concentrations and with or without 100µM apyrase-treated ApppI. OD₃₄₀ₙₘ was measured during 2 minutes (with one measurement every 10 seconds) on a Varioskan Flash spectrophotometer (Thermo Electron Corporation). The initial hydrolysis rate (V₀) was calculated from a linear regression of the data. During this lapse time the recording of optical density invariably produced linear curves.

### Single cell analysis of calcium mobilization

Vγ9Vδ2 T cells were loaded with fura-2-AM (Molecular probes, 5µM, 20min) in 1mL of RPMI, washed, and 10⁵ cells were left to adhere for 10min to the bottom of polylysin-D-treated (5µg/ml) 8-well Labtek chamber slides in 100µL of RPMI containing 5% FCS and 10mM Hepes. Fluorescence measurements were performed on a Zeiss Axiovert 200M inverted microscope equipped with a CCD camera and a 37°C, 5% CO₂ chamber. Cells were consecutively excited at 340 and 380nm at intervals of 10s and emission at 510nm was collected with the CCD camera in turn with transmitted light images. Analysis was performed on individual cells which did not make contacts with surrounding cells during the time of recording. After initial focusing, acquisition was paused and antigens and beads were added in a volume of 20µL deposited on the top of the cell medium. It took 2-3min for the beads to come in contact with the adherent T cells. The camera output was analyzed with the Metafluor software. Data were transferred into the Excel software for conversion of fluorescence ratios 340nm/380nm to intracellular calcium concentrations and averaging. Intracellular calcium concentrations were calculated as described (Grynkiewicz, G. et al. J Biol Chem 260, 3440-3450, 1985) and using ionomycin stimulation (5nM) to calculate the maximum ratio.

### RESULTS

Vγ9Vδ2 lymphocytes are activated by soluble pyrophosphorylated antigens such as IPP or by tumor cells overproducing it. IPP gives rise to the nucleosidic metabolite ApppI and we show that ApppI represents an inactive storage form of phosphoantigen which requires conversion into IPP for activity. ApppI can be pulsed on tumors to confer a stimulatory activity resistant to phosphatases, indicating that the antigen has been processed or protected. Tumor recognition also involves TCR binding to the ecto-F₁-ATPase complex. ApppI can stably bind to F1-ATPase immobilized on beads and the stimulatory activity can be released by exogenous addition of nucleotide pyrophosphatase. Single cell analysis of T cells reveals that phosphoantigens and F₁-ATPase cooperate to deliver the signal for TCR activation.

### Direct stimulation of γδ T cells with nucleosidic phosphoantigens

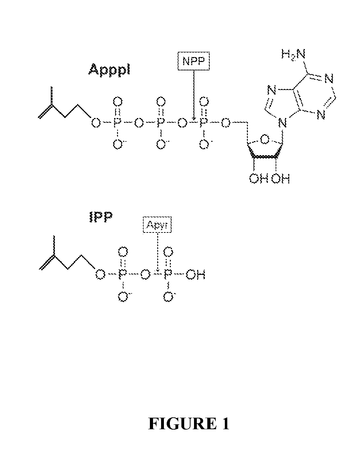

Studies by Morita et al. indicated that addition of nucleosides to monoethyl pyrophosphate induce little change in the stimulatory activity for Vγ9Vδ2 T cells (Morita, C.T. et al. J Immunol 167, 36-41, 2001). We compared here the bioactivity of IPP, a phosphoantigen overproduced in tumors, and a synthetic adenylated compound, (1-(adenosin-5'-yl) 3-(3-methylbut-3-en-1-C14-yl) triphosphoric diester) which will be referred to as ApppI: in this compound the isopentenyl moiety is linked to the γ-phosphate of ATP instead of being linked to a pyrophosphate group (Fig.1). This compound is also overproduced in tumors after stimulation with aminobisphosphonates (Monkkonen, H. et al. Br J Pharmacol 147, 437-445, 2006).

When added in a culture of peripheral blood lymphocytes (PBL), ApppI induces the selective expansion of Vγ9Vδ2 T cells, so that the culture reaches homogeneity (∼94%) in 3 weeks, as it is the case for most phosphoantigens (Fig.2a). IPP and ApppI could induce proliferation and intracellular IFN-γ production. However, ApppI required a 5 to 10-fold higher molar concentration for a similar effect on Vγ9Vδ2 T cell expansion from PBLs and a 50 to 100-fold higher concentration for a similar cytokine response (Fig.2b and 2c). Both compounds also induced the cytotoxicity machinery as evidenced by the surface expression of the lysosomal marker CD107a although IPP induced again a stronger stimulation (Fig.2e). Thus the adenylated derivative of IPP is less bioactive than IPP itself for Vγ9Vδ2 T cell stimulation. Addition of ApppI to IPP tends to increase stimulation, indicating that ApppI does not compete with IPP (Fig.2d).
The structure of ApppI indicates that it could be converted to IPP + AMP through cleavage of the α-β phosphoester bond by a nucleotide pyrophosphatase activity (Bollen, M. et al. Critical reviews in biochemistry and molecular biology 35, 393-432, 2000). In order to test for this possibility, a Vγ9Vδ2 T cell line was stimulated with ApppI in the presence or absence of exogenous crotale venom nucleotide pyrophosphatase (NPP). When this enzyme was added to T cell stimulation assays it greatly increased the stimulatory activity of ApppI which even exceeded IPP response obtained with the same molar dose. Strikingly, NPP invariably increased also IPP response although the enzyme was deprived of any effect in the absence of antigen. In the presence of the enzyme, ApppI and IPP displayed the same bioactivity. This indicates that in cell cultures a part of IPP is converted into a less active nucleoside derivative (Fig. 3a).
The phosphate groups of IPP are sensitive to terminal phosphatases such as alkaline phosphatase (Constant, P. et al. Science 264, 267-270, 1994; Tanaka, Y. et al. Nature 375, 155-158, 1995) or apyrase. Conversely ApppI is expected to be resistant to degradation by the same enzymes. Indeed, treatment of ApppI with apyrase did not change its HPLC profile whereas ATP is completely hydrolyzed in similar conditions (Fig.4). On the contrary, ApppI can be hydrolyzed by NPP. In order to explore a possible interconversion between IPP and ApppI, we tested the effect of apyrase and NPP when added in T cell stimulation assays (Fig.3b). As expected from previous reports, IPP stimulatory activity is completely abrogated by addition of apyrase, indicating that the terminal phosphates are required for T cell activation. Strikingly, apyrase also abrogates completely the stimulation by ApppI indicating that T cell activation by this compound is due to its prior conversion into a compound in which the phosphate is terminal, most likely IPP. Indeed, the HPLC profile of NPP-treated ApppI indicates the release of a moiety which is presumably AMP and is compatible with the expected cleavage of ApppI between the α and β phosphates (relative to the adenosinyl moiety) since it does not lead to release of ADP (Fig.4). The complete abrogation of ApppI activity by addition of apyrase in the stimulation assay also indicates that ApppI is not stimulatory by itself and requires conversion into IPP by a pyrophosphatase activity present in the cell culture medium. This is confirmed by the inhibition of ApppI stimulatory activity by addition of the nucleotide pyrophosphatase inhibitor pyridoxal phosphate-6-azophenyl-2',4'-disulfonic acid (PPADS, Grobben, B. et al. J Neurochem 72, 826-834, 1999) (Fig.3c). PPADS also partially decreases IPP stimulatory activity. This can be explained by the partial conversion of IPP into ApppI and possible rescue of IPP by a nucleotide pyrophosphatase activity present in the medium. Altogether, these data strongly support an interconversion equilibrium between IPP and ApppI in the culture medium, IPP being the stimulatory entity for the direct stimulation of Vγ9Vδ2 T cells, whereas ApppI has no intrinsic stimulatory activity. However, as ApppI can be converted into IPP, it might represent a storage form of phosphoantigen which is resistant to ubiquitous phosphatases.

### Effect of APCs on phosphoantigen-mediated stimulation

It has been widely documented that Vγ9Vδ2 T cell stimulation is increased by the presence of bystander APCs although phosphoantigens cannot be pulsed efficiently on cells (Lang, F. et al. J Immunol 154, 5986-5994 (1995).
We investigated the effect of APCs in the case of ApppI-mediated stimulation in comparison to IPP stimulation. RPMI-8226 myeloma cell line has weak stimulatory activity for Vγ9Vδ2 T cells (Selin, L.K., et al. Scand J Immunol 36, 107-117, 1992) and was used as APC. Stimulation of a Vγ9Vδ2 T cell line by a combination of RPMI-8226 cells and IPP or ApppI results in a synergistic activation as the resulting stimulation exceeds the sum of both stimulations (Fig.5a). However this effect appeared surprisingly more pronounced in the case of ApppI. This prompted us to evaluate the possibility that the response to ApppI could involve presentation by tumor cells. Different tumor cell lines were pulsed overnight with IPP or ApppI, extensively washed and used as stimulating cells for Vγ9Vδ2 T cells. As previously reported for classical non-nucleosidic phosphoantigens, pulsing of tumors with IPP is poorly efficient and less than 20% of the activity is retained after extensive washing (Fig.5b, closed bars). By contrast, pulsing of the same tumor lines with ApppI unexpectedly confers an increased stimulatory activity which is retained after extensive washing. Indeed, 70% of the stimulatory activity of ApppI is retained on cells as compared to when tumor cells are not washed. As a result, cells which have been pulsed with ApppI are potent stimulators in contrast to cells pulsed with the same molar concentration of IPP. Strikingly, 721.221 cell line cannot be significantly pulsed with ApppI showing that susceptibility to pulsing by ApppI is not a general property of human cells.

### ApppI-pulsed cells behave as classical Vγ9Vδ2 target cell lines

Daudi cells strongly stimulate Vγ9Vδ2 lymphocytes. This is likely due to overstimulation of the mevalonate pathway and production of endogenous phosphoantigens. A similar stimulation can be obtained with most human cell lines treated with aminobisphosphonates which inhibit farnesyl pyrophosphate synthase and induce an accumulation of IPP in cells. Conversely, treatment of Daudi cells with statins which inhibit HMG-CoA reductase abrogates the spontaneous stimulatory activity of these cells (Gober, H.J. et al. J Exp Med 197, 163-168, 2003). We hypothesized that ApppI could possibly act on the mevalonate pathway and also lead to IPP accumulation. If this were the case, the activity of ApppI pulsed cells would be abrogated by treatment of the cells with statins. Fig.5d indicates that treatment of K562 cells with lovastatin does not inhibit the stimulatory activity induced by pulsing with ApppI whereas it efficiently abrogates stimulatory activity induced by pamidronate (an aminobisphosphonate). Thus ApppI and pamidronate act differently on cells.
As the direct stimulation of Vγ9Vδ2 T cells by ApppI can be modulated by addition of pyrophosphatase and apyrase, it was important to check the effect of such treatment on the stimulation by pulsed cells. These enzymes were thus added separately or in combination in T cell stimulation assays using either Daudi or K562 cells pulsed with pamidronate or ApppI. It surprisingly appears (Fig.5) that such stimulation is completely resistant to treatment with apyrase and pyrophosphatase either alone or in combination. Thus the antigen, whether it is IPP or ApppI, is somehow processed to become resistant to these enzymes and is stably presented on the surface of tumors. This also indicates that the Vγ9Vδ2 T cell stimulation by antigen-pulsed tumors and their direct stimulation by soluble IPP result from different mechanisms and different antigenic forms.
As ApppI can be pulsed on tumor cells, we investigated whether this resulted from passive adsorption on the cell surface or required an active processing mechanism. To study this mechanism we used short pulsing conditions (2h) to prevent cell alteration. Two hours of pulsing with ApppI are sufficient to confer stable stimulatory activity to RPMI-8226 cells. However, pulsing is abrogated when performed at 4°C or on cells which were previously treated with the actin polymerization inhibitor cytochalasin D (Fig.5c). This indicates that pulsing requires an active mechanism and involves cytoskeletal components, indicative of an active process.

### ApppI is a ligand for F₁-ATPase

Previous studies have demonstrated that ecto-F₁-ATPase is expressed on the surface of tumors which stimulate Vγ9Vδ2 T cells (Scotet, E. et al. Immunity 22, 71-80, 2005). Moreover, F₁-ATPase is a ligand for the Vγ9Vδ2 TCR and in some conditions can promote Vγ9Vδ2 T cell activation. 721.221 cells remain poorly stimulatory for Vγ9Vδ2 T cells even after pulsing with pamidronate (not shown) or ApppI (Fig.5b). Moreover, extensive immunofluorescence and biochemistry experiments failed to demonstrate the presence of ecto-F₁-ATPase complex on these cells. It is thus tempting to hypothesize that ecto-F₁-ATPase is somehow involved in phosphoantigen presentation on the surface of tumors. Indeed, this complex can bind adenine nucleotides such as ATP, ADP and other nucleotides (Noji, H. et al. J Biol Chem 276, 25480-25486, 2001; Perlin, D.S. et al. Biochemistry 23, 4998-5003, 1984; Allison, W.S. et al. J Bioenerg Biomembr 24, 469-477, 1992). In a first attempt to comfort this hypothesis, we analyzed the putative effect of ApppI on the enzymatic activity of F₁-ATPase purified from bovine heart mitochondria. ATP hydrolysis was measured in an enzymatic bioluminescence assay for ATP and a photo colorimetric assay for ADP production. In both assays, we observed that addition of ApppI increased ATP hydrolysis to form ADP. The amount of ATP which is hydrolyzed by the complex is increased by two-fold when ApppI is added in the reaction mixture. The enzymatic kinetics of ADP production indicates that the initial rate of ADP production at low ATP concentrations in the presence of 100 µM ApppI is approximately twice the rate of ADP production in the absence of ApppI. Thus ApppI stimulates F₁-ATPase activity indicating that it is a possible ligand for the complex (Fig.6).
We next investigated on the possibility that ApppI could stably bind to F1-ATPase. To this end, bovine F₁-ATPase (in the form of F₁Fₒ complexes) was immobilized on polystyrene beads. The beads were then incubated with ApppI, extensively washed and tested for stimulatory activity on Vγ9Vδ2 T cells (Fig.7). F₁-ATPase has a low basal stimulatory activity for Vγ9Vδ2 T cells. Loading of ApppI onto F₁-ATPase-coated beads confers some additional stimulatory activity to the complex. However, when NPP is added in the T cell stimulation assay, a strong stimulatory activity can be revealed, indicating that ApppI has bound to F₁-ATPase and that stimulatory IPP can be released from the beads provided that an NPP activity is present. If the F₁-coated beads loaded with ApppI are treated with NPP after pulsing, washed and co-incubated with T cells, they retain an activity identical to that of beads which have not been treated with NPP, indicating that once bound to F₁, ApppI is protected from NPP hydrolysis. By contrast, when ApppI is treated with NPP before pulsing on beads, no activity is retained, indicating that the antigen must be in the form of a nucleotide conjugate for binding stably to F₁. In accordance, IPP was not retained on beads and no activity could be recovered with or without NPP. Exogenous addition of NPP strongly increases the stimulatory activity of ApppI-pulsed F₁ when it is added during the stimulation. This indicates that ApppI becomes susceptible to NPP hydrolysis upon interaction with T cells. A possible explanation is that T cell contact promotes the release of the antigen. Released ApppI would then be hydrolyzed by an NPP activity which is present in the T cell environment and is further increased by exogenous addition of the enzyme. A similar phenomenon could occur on the tumor cell surface since NPP also tends to increase the stimulatory activity of Daudi cells (Fig.5e).

Published experiments have shown that "direct" T cell activation by phosphoantigens requires cell contact with bystander sister cells suggesting the involvement of some mandatory presentation or co-stimulation mechanism (Morita, C.T. et al., Immunol Rev 215, 59-76, 2007). We thus set up an experiment to determine whether ecto-F₁-ATPase could be involved in this mechanism. Living Vγ9Vδ2 T cells were layered on microscopy slides in conditions where cell-cell contact was avoided. T cell activation was monitored by measuring the intracellular calcium mobilization using wide field fluorescence microscopy after addition of different antigens in the presence or absence of an excess of F₁-ATPase immobilized on polystyrene beads (Fig.8). As previously reported (Morita, C.T. et al. Immunity 3, 495-507, 1995), IPP alone or in presence of control beads promoted no calcium flux in cells which did no make additional contacts although some possible "receptor teasing" resulted in a spiky pattern of activation in rare cells. Addition of F₁-coated beads in the absence of IPP induced a borderline increase of intracellular calcium in few cells. However, addition of F₁-coated beads on cells in the presence of IPP promoted a progressively increasing and sustained calcium flux starting after contact with F₁-ATPase revealing that T cell interaction with F₁ synergizes with IPP and can compensate for cell-cell contact. Intracellular calcium concentration was still increasing at the end of the recording (90min). This slow response contrasted with the rapid response to IPP in T cells making contacts with other cells (Morita, C.T. et al., Immunol Rev 215, 59-76, 2007 and data not shown) and may be due the fact that T cells establish very short-lasting contacts with rapidly moving beads as these did not adhere strongly to the bottom of the wells nor to cells. ApppI-loaded, F₁-coated beads promoted a clear activation in few cells only although again a possible receptor teasing resulted in the formation of initial calcium "spikes". When NPP was added after contact with ApppI-loaded, F₁-coated beads, this resulted in a slow but strong and sustained calcium response indicating that active phosphoantigen is not released spontaneously from F₁ and requires exogenous addition of pyrophosphatase in this experimental setting. These results indicate that F₁-ATPase can act as a presenting molecule for nucleosidic antigens and provides a co-stimulatory signal for the response to the pyrophosphate metabolite once it is released.

### Conclusions

Whereas studies emphasized the fact that phosphoantigens cannot be pulsed on presenting cells (Tanaka, Y. et al. Proc Natl Acad Sci U S A 91, 8175-8179, 1994; Morita, C.T. et al. Immunity 3, 495-507, 1995), we now show that it is clearly not true for nucleosidic phosphoantigens, notably ApppI. Our data evidence that nucleosidic phosphoantigens can be efficiently pulsed on cells and reveal the existence of an antigen processing and presentation mechanism for phosphoantigens which is reminiscent of peptide processing for MHC presentation or lipid processing for loading on CD1 molecules. Without wanting to be bound by any theory, we think that, as F₁-ATPase can bind the TCR and the nucleosidic antigen, it may provide a mean to deliver the antigen in the proximity of the TCR and to keep this antigen away from surrounding phosphatases until a pyrophosphatase (certainly an ecto-pyrophosphatase present on the cell surface, notably on a tumor cell surface, see Goding, J.W. et al. Immunol Rev 161, 11-26, 1998), comes into play and releases the active phosphoantigen (IPP in case of ApppI). The phosphoantigen would then be able to stimulate the TCR independently of ecto-F₁-ATPase binding. However, single cell stimulation experiments (Fig.8) indicate that F₁-ATPase also provides an additional stimulus which can replace the otherwise required cell-cell contact. We thus favour a scenario in which F₁-ATPase actively participates to the stimulation mechanism in synergy with the phosphoantigen. Finally, our data showing that nucleosidic phosphoantigen pulsing on tumor cells is abrogated by cytochalasin D suggests that an active internalization process is involved in phosphoantigen display at the cell surface when these antigens are provided exogenously.

Altogether we have shown the existence of a new processing and presentation mechanism for nucleosidic phosphoantigens which reconciles Vγ9Vδ2 T cell biology with that of other T cell populations, and offered a new method for stimulating Vγ9Vδ2 T cell-mediated immune response.

### REFERENCES

Throughout this application, various references describe the state of the art to which this invention pertains. The disclosures of these references are hereby incorporated by reference into the present disclosure.

## Claims

1. A compound of general formula (I) for the treatment of the human or animal body by therapy: 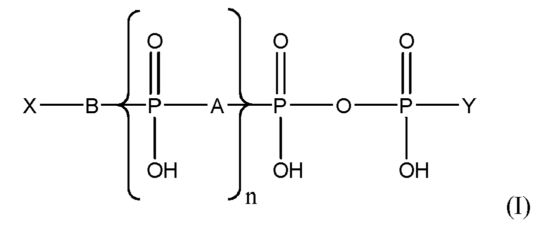 wherein
- n is an integer from 1 to 5;
- B is present or not and if present is selected from the group consisting of O, NH, CHF, CF₂ and CH₂;
- Y is a nucleoside;
- X is a linear or branched C₁-C₅ alkyl or C₂-C₅ alkylenyl, substituted or not by at least one halogen atom, an hydroxyl group or an epoxide group; and
- if n is 1, A is present or not and if present is selected from the group consisting of O, NH, CHF, CF₂ and CH₂; and
- if n is 2, 3, 4 or 5, A is independently present or not and if present is independently selected from the group consisting of O, NH, CHF, CF₂ and CH₂,
or a pharmaceutically acceptable salt thereof,
wherein said compound
i) is cleavable by a nucleotide pyrophosphatase, and
ii) can bind to F1-ATPase.

2. A compound according to claim 1, wherein
- n is from 1 to 3;
- B is selected from the group consisting of O, NH, CHF, CF₂ and CH₂;
- Y is a nucleoside;
- X is a linear or branched C₂-C₅ alkylenyl substituted or not by at least one halogen atom, an hydroxyl group or an epoxide group; and
- A is O,
or a pharmaceutically acceptable salt thereof.

3. A compound according to claim 1, wherein said compound is ApppI or a pharmaceutically acceptable salt thereof.

4. A compound according to any one of claims 1 to 3, wherein said compound is for stimulating Vγ9Vδ2 T cell-mediated immune response.

5. A compound according to any one of claims 1 to 3, wherein said compound is for treating a disease selected for the group consisting of cancers, infectious diseases, autoimmune diseases and inflammatory diseases.

6. A compound according to claim 5, wherein said compound is for treating an autoimmune disease or an inflammatory disease.

7. A method for pulsing human antigen-presenting cells with a compound as defined in any of claims 1 to 3, wherein said method comprises the step of culturing antigen-presenting cells *ex vivo* with at least one of a compound as defined in any of claims 1 to 3.

8. An antigen-presenting cell obtainable by the method of claim 7, for the treatment of the human or animal body by therapy.

9. An antigen-presenting cell according to claim 8, for treating a disease selected for the group consisting of cancers, infectious diseases, autoimmune diseases and inflammatory diseases.

10. A vaccine comprising an antigen or a combination of antigens and a compound as defined in any of claims 1 to 3.

11. A vaccine according to claim 10, for treating a disease selected from the group consisting of cancers and an infectious diseases.

12. A vaccine kit comprising
a) an antigen or a combination of antigens; and
b) a compound as defined in any of claims 1 to 3.

13. A compound according to claim 5 or an antigen-presenting cell according to claim 9 or a vaccine according to claim 11, wherein said disease is a cancer.

14. A compound according to claim 13 or an antigen-presenting cell according to claim 13 or a vaccine according to claim 13, wherein said cancer comprises tumor cells expressing on their surface the F₁-ATPase.

15. A compound according to claim 5 or an antigen-presenting cell according to claim 9 or a vaccine according to claim 11, wherein said disease is an infectious disease.
